Europäisches Patentamt

European Patent Office

Office européen des brevets

(11) Veröffentlichungsnummer: **0 142 759**
**A2**

(12) # EUROPÄISCHE PATENTANMELDUNG

(21) Anmeldenummer: **84113255.8**

(22) Anmeldetag: **03.11.84**

(51) Int. Cl.⁴: **A 61 F 2/34**

(30) Priorität: **22.11.83 DE 3342035**

(43) Veröffentlichungstag der Anmeldung:
**29.05.85 Patentblatt 85/22**

(84) Benannte Vertragsstaaten:
**AT BE CH DE FR GB IT LI NL SE**

(71) Anmelder: **Protek AG**
**Stadtbachstrasse 64**
**CH-3012 Bern(CH)**

(72) Erfinder: **Weill, Dan, Dr.**
**Château de buy Antilly**
**F-57640 Vigy(FR)**

(74) Vertreter: **Lusuardi, Werther Giovanni, Dr.**
**Lyss-Strasse 12**
**CH-3293 Dotzigen(CH)**

(54) **Hüftgelenkpfanne.**

(57) Die Hüftgelenkspfanne besteht aus einem Kunststoff-Pfannenkörper (1) und einem diesen umgebenden Ring (8) aus Metall. Beide Einzelelemente sind über einen Aussenkonus (6) und einen Innenkonus (7) im Passsitz miteinander verbunden. Dabei rasten Ansätze (10) in Ausnehmungen (11) des Ringes (8) ein und sind durch seitliche Schrägflächen (13) an den Ansätzen (10) bzw. Hinterschneidungen (14) in den Ausnehmungen (11) gegen ein Herausfallen gesichert.

Die neue Pfanne ist bei Kaltfliessen des Kunststoffes selbstfixierend, so dass zu Abrieb führende dauernde Relativbewegungen zwischen Ring (8) und Pfannenkörper (1) vermieden werden.

Fig. 2

EP 0 142 759 A2

Hüftgelenkspfanne

Die Erfindung betrifft eine Hüftgelenkspfanne zur zementfreien Verankerung im Becken, bestehend aus einem starren Aussenkörper und aus einem in diesen eingelegten Pfannenkörper.

Bei Implantaten für den menschlichen Körper ist man ganz allgemein bestrebt, den Fremdkörper im Gewebe möglichst klein im Volumen zu halten. Dieses Bestreben führt bei künstlichen Gelenkpfannen zu relativ dünnwandigen Pfannenkörpern; da wegen der Gleiteigenschaften die Pfannenkörper sehr häufig aus Kunststoff, insbesondere aus Polyäthylen, hergestellt werden, sind die Pfannenkörper relativ nachgiebig und übertragen die Beckenbewegungen auf die zueinander kongruenten Gleitflächen. Diese Bewegungen führen zu dem sogenannten "Kirschkern"-Effekt, worunter man das "Herausarbeiten" eines festen Kerns aus einem weicheren Material bei Relativbewegungen zueinander versteht. Sofern die Pfannenkörper aus Kunststoff bestehen, neigen sie unter den dauernden Belastungen weiterhin zu plastischen Verformungen, die zu Veränderungen ihrer Schalenfläche führen. Dadurch wird die Kongruenz der eine Gleitfläche des Gelenks bildenden Schalenoberfläche mit der anderen Gleitfläche, die von der Oberfläche des Gelenkkopfes gebildet wird, gestört, was zu erhöhtem Abrieb führt.

Es besteht daher die Forderung, dass die Hüftgelenkspfanne als Ganzes möglichst fest und starr sein soll, so dass sie

0142759

sich bei Bewegungen des Gelenkes nicht verformt. Diese Forderung hat zu einer Anzahl von Konstruktionen geführt, bei denen ein Pfannenkörper von einem Aussenkörper umhüllt und in diesem gehalten wird (siehe z.B. DE-OS 29 50 536). Damit wird zwar ein Pfannenkörper gegen den direkten Einfluss der Beckenbewegungen geschützt; bei diesen Konstruktionen treten aber - besonders beim plastischen Kaltfliessen eines Pfannenkörpers aus Kunststoff - Relativbewegungen zwischen dem Pfannenkörper und seinem Aussenkörper auf, die zu Verschleiss und Abrieb führen, der zwischen die beiden Gleitflächen gelangen kann. Weiterhin stellt ein den Pfannenkörper umhüllender, schalenförmiger Aussenkörper eine grosse Fremdmasse dar.

Aufgabe der Erfindung ist es, eine starre, konische Hüftgelenkspfanne möglichst geringen Volumens zu schaffen, die gegen die Beckenbewegungen widerstandsfähig ist, die Gleiteigenschaften von Kunststoffpfannen besitzt und sich bei plastischen Verformungen des Pfannenkörpers wieder selbsttätig fixiert.

Diese Aufgabe wird erfindungsgemäss dadurch gelöst, dass der sich gegen kranial verjüngende, mit Rippen versehene Aussenkörper einen offenen Ring mit einem inneren Hohlkonus für einen daran drehfest angepassten Konus des Pfannenkörpers bildet.

Der im allgemeinen aus Kunststoff gefertigte Pfannenkörper wird durch den Ring auf seinem Mantel so versteift, dass sich eine starre und feste Pfanne ergibt. Die Ringform des Aussenkörpers reduziert das Fremdkörpervolumen im Gewebe - bei gleichbleibender Pfannengrösse - gegenüber der bekannten Konstruktion entscheidend. Darüberhinaus wird dadurch in Verbindung mit der konischen Innenform beim Fliessen des Kunststoffes eine Selbstfixierung erreicht.

In einer weiteren Ausgestaltung der Erfindung sind über den Umfang verteilt in der Stirnfläche des Ringes mindestens drei, mit schrägen Hinterschneidungen versehene Ausnehmungen vorgesehen, in die mit zu den Hinterschneidungen gleichlaufenden Schrägflächen versehene Ansätze des Pfannenkörpers eingreifen. Die schrägen Hinterschneidungen ermöglichen eine einfache Montage des Pfannenkörpers in dem Ring, der bei der Implantation zuerst in das vorbereitete Becken eingeschlagen oder eingeschraubt wird.

Die Selbstfixierung des Pfannenkörpers nach plastischen Verformungen kann erleichtert werden, wenn in axialer Richtung zwischen der Stirnfläche des Ringes und dem Boden der Ansätze ein Spiel verbleibt.

Ein weiterer Vorteil der Ringform des Aussenkörpers besteht darin, dass dadurch ein gleichzeitiges Aufliegen von Seitenflanken und Boden vermieden wird, was infolge unvermeidbarer Toleranzen zu einer statischen Ueberbestimmung des eingesetzten Aussenkörpers führt. Eine solche Ueberbestimmung bewirkt, dass bei aufliegendem Boden an den Seitenflächen keine Klemmwirkung mehr erreicht wird.

Die einfache Montage mit Hilfe von vorzugsweise vier schrägen Hinterschneidungen schafft die Möglichkeit, während der Implantation durch probeweises Einsetzen mehrerer Pfannenkörper mit unterschiedlichen Pfannenschalengrössen den geeignetsten auszuwählen.

Während der Pfannenkörper im allgemeinen aus Kunststoff, vorzugsweise Polyäthylen, besteht, ist das Material für den Ring ein in der Implantat-Technik übliches Metall oder eine Metall-Legierung, vorzugsweise Reintitan.

Zur sicheren Verankerung der Hüftgelenkspfanne im Becken sind die Rippen des Aussenkörpers mit Vorteil als zweigängiges Lamellengewinde mit einer Steigung von 4-6 mm, vorzugsweise von 4,5 - 5,5 mm und einer Lamellenhöhe von 2 bis 4 mm, vorzugsweise von 2,5 bis 3,5 mm ausgebildet, welches mit axialen Schneidnuten versehen werden kann, um das Gewinde selbstschneidend zu machen.

Die Aussenform des Aussenkörpers ist vorteilhafterweise kugelkalottenförmig ausgebildet.

Im folgenden wird die Erfindung anhand eines Ausführungsbeispiels im Zusammenhang mit der Zeichnung näher erläutert.

Fig. 1 zeigt eine Aufsicht auf die Aequatorebene
der neuen Hüftgelenkspfanne in Richtung
der Achse ihrer konischen Form;

Fig. 2 ist ein Schnitt II-II von Fig. 1;

Fig. 3 gibt in grösserem Massstab eine Ansicht
von Fig. 1 in Richtung des Pfeiles A wieder.

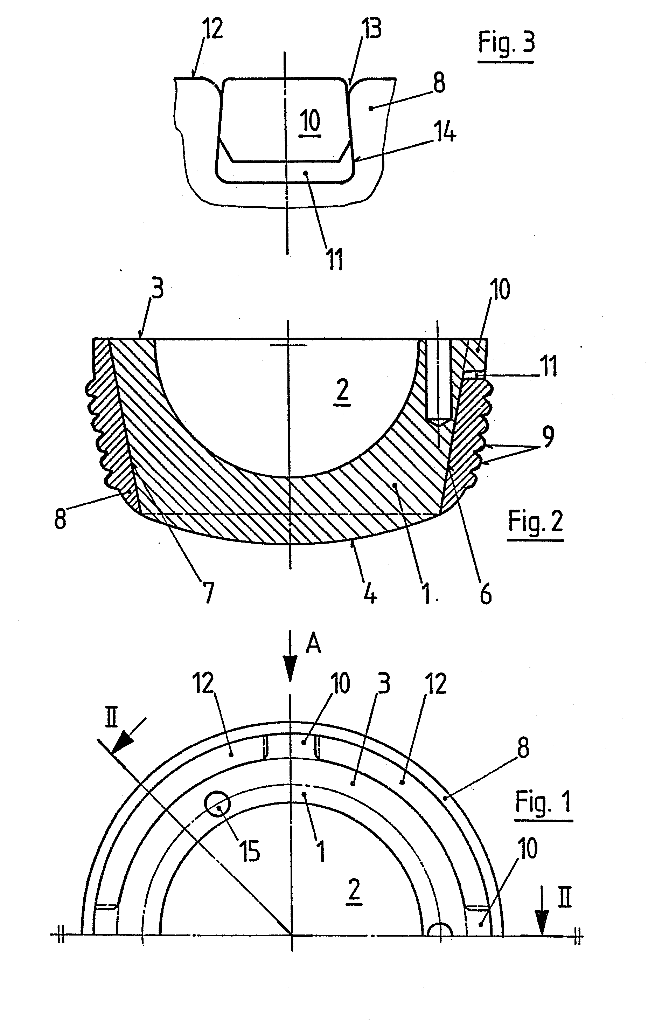

Die Hüftgelenkspfanne nach Fig. 2 hat einen Pfannenkörper 1,
dessen Querschnitt ein gleichseitiges Trapez mit gewölbtem
Boden 4 bildet, so dass seine Grundform ein Kegelstumpf ist.
Von der breiten Basis 3 aus ist in den Pfannenkörper 1 die
eigentliche Pfannenschale 2 in Form einer hohlen Halbkugel
eingearbeitet, die den Gelenkkopf einer nicht dargestellten
Femurkopfprothese aufnimmt.

Auf dem Konus 6 des Pfannenkörpers 1 sitzt ein innerer
Hohlkonus oder Innenkonus 7 eines Metallrings 8, der auf
seiner Aussenseite mit einer Verankerungsstruktur 9, beispielsweise mit Tragrippen oder einem Schraubengewinde,
versehen ist.

Der Konus 6 des Pfannenkörpers 1 und der Innenkonus 7 des
Ringes 8 sind so aufeinander abgestimmt, dass sie im Passsitz ineinandergreifen.

Wie aus Fig. 1 zu ersehen ist, hat der Pfannenkörper an
der Basis 3 vier um je 90$^{\circ}$ zueinander versetzte Ansätze 10,
die in entsprechende Ausnehmungen 11 in der Stirnfläche 12
des Ringes 8 einrasten. Wie weiterhin Fig. 3 zeigt, sind
die Seitenflanken der Ansätze 10 und der Ausnehmungen 11

0142759

mit sich von der Stirnfläche 12 des Ringes 8 weg erweiternden Schrägflächen 13 bzw. Hinterschneidungen 14
versehen, die dazu dienen, ein Herausfallen des Pfannenkörpers 1 aus dem Ring 8 zu verhindern.

Weiterhin sind die Konen 6 und 7, die Tiefe der Ausnehmungen 11 und die Höhe der Ansätze 10 so aufeinander abgestimmt,
dass zwischen den Ansätzen 10 und dem Boden der Ausnehmung 11
jeweils ein Spiel vorhanden ist, so dass der Pfannenkörper 1
bei plastischen Verformungen tiefer in den Innen- oder
Hohlkonus 7 eindringen kann, ohne dass es in den Ausnehmungen 11 zu Verformungen an den Ansätzen 10 kommt.

Schliesslich sind im Pfannenkörper 1 drei Bohrungen 15 für
den Einsatz eines Instrumentes vorgesehen.

Patentansprüche

1. Hüftgelenkpfanne zur zementfreien Verankerung im Becken, bestehend aus einem starren Aussenkörper und aus einem in diesen eingelegten Pfannenkörper (1), dadurch gekennzeichnet,dass der sich gegen kranial verjüngende mit Rippen (9) versehene Aussenkörper einen offenen Ring (8) mit einem inneren Hohlkonus (7) für einen daran drehfest angepassten Konus (6) des Pfannenkörpers (1) bildet.

2. Hüftgelenkspfanne nach Anspruch 1, dadurch gekennzeichnet, dass über den Umfang verteilt in der Stirnfläche (12) des Ringes (8) mindestens drei, mit schrägen Hinterschneidungen (14) versehene Ausnehmungen (11) vorgesehen sind, in die mit zu den Hinterschneidungen (14) gleichlaufenden Schrägflächen (13) versehene Ansätze (10) des Pfannenkörpers (1) eingreifen.

3. Hüftgelenkspfanne nach Anspruch 2, dadurch gekennzeichnet, dass in axialer Richtung zwischen den Ausnehmungen (11) des Ringes (8) und dem Boden der Ansätze (10) ein Spiel verbleibt.

4. Hüftgelenkspfanne nach einem der Ansprüche 2 oder 3, dadurch gekennzeichnet, dass vier Ausnehmungen (11) vorgesehen sind.

5. Hüftgelenkspfanne nach einem der Ansprüche 1 bis 4, dadurch gekennzeichnet,dass der Aussenkörper aus Reintitan und der Pfannenkörper (1) aus Polyäthylen besteht.

6.Hüftgelenkspfanne nach einem der Ansprüche 1 bis 5 dadurch gekennzeichnet,dass die Aussenform des Aussenkörpers kugelkalottenförmig ausgebildet ist.

7. Hüftgelenkpfanne nach einem der Ansprüche 1 bis 6, dadurch gekennzeichnet, dass die Rippen des Aussenkörpers als zweigängiges Lamellengewinde ausgebildet sind.

8. Hüftgelenkspfanne nach Anspruch 7, dadurch gekennzeichnet, dass das Lamellengewinde eine Steigung von 4-6mm vorzugsweise von 4,5 bis 5,5mm aufweist.

9. Hüftgelenkspfanne nach Anspruch 7 oder 8, dadurch gekennzeichnet,dass die Lamellenhöhe 2 bis 4mm, vorzugsweise 2,5
bis 3,5mm beträgt.

10. Hüftgelenkspfanne nach einem der Ansprüche 7 bis 9, dadurch gekennzeichnet, dass das Lamellengewinde mit axialen
Schneidnuten versehen ist.

0142759

Fig. 3

12  13  8

10

14

11

3  10

11

2

9

8

7  4  1  6

Fig. 2

A

12  10  3  12

8

II

15  1

2

10

II

Fig. 1